# EUROPEAN PATENT APPLICATION

(11) **EP 3 031 895 A1**
(43) Date of publication of application: **15.06.2016**
(21) Application number: 14382511.5
(22) Date of filing: 12.12.2014
(51) Int. Cl.: C12M 1/00, C12M 1/06

(54) **Aeration and agitation system for the culture of microorganisms in single use bioreactors**

(71) Applicant: Universitat Autònoma de Barcelona, 08193 Bellaterra (Cerdanyola del Valles) (ES)
(72) Inventor: Lecina Veciana, Martí, 08193 Bellaterra (Cerdanyola del Vallès) (ES); Cairó Badillo, Jordi Joan, 08193 Bellaterra (Cerdanyola del Vallès) (ES); Casablancas Mira, Antoni, 08193 Bellaterra (Cerdanyola del Vallès) (ES); Martinez Monge, Ivan, 08193 Bellaterra (Cerdanyola del Vallès) (ES); Paredes Muñoz, Carlos J., 08193 Bellaterra (Cerdanyola del Vallès) (ES)

(57) **Abstract**

The present invention describes a device for improving the oxygen transfer in single use bioreactors thanks to an agitation-aeration system generating microbubbles with pore sizes ranging from 0,5 microns up to 1 mm. The microbubble generating system might be coupled to the agitation shaft, be independent of it or a combination of both simultaneously. This invention opens the door to the use of 2-2000 liters single use plastic bioreactors for the culture of different biological specimens (bacteria, yeasts, fungi, animal and plant cells) using a single type of bioreactor.

## Description

### Introduction

The use of single use bioreactors (SUB) for animal cell culture at scales between 2 and 2000 liters has been already described (Fenge C, Lüllau E. Cell Culture Biorreactors for Pharmaceutical and Cell Based Therapies. Ozturk S, HU WS, Eds. Taylor and Francis Group: Oxford, UK. 2006; 155-224). The applications of the substances produced through animal cell culture span the fields of biomedicine, biotechnology, food industry and environment. However, there are equally interesting substances produced through the cultivation of bacteria, yeasts and unicellular fungi (mainly aerobic, but also facultative and anaerobic). Unfortunately, currently available SUBs cannot achieve industrially relevant cell densities when using those organisms due their poor oxygen transfer rate between the gas and the liquid phases.

From a production point of view, SUB have the following advantages over stainless steel bioreactors: lower fixed investments in equipment and auxiliary equipment, reduced needs of continuous cycles of cleaning and sterilization and their verification in accordance with GMP standards and, as a consequence, reduced manpower requirements (Sinclaire A, Monge M. Quantitative Economic Evaluation of Single-use Disposables in Bioprocessing. Pharmaceutical Engineering, 2002:20-34). Moreover, the rapid transition between processes enabled by single use systems increases productivity thanks through a reduced turnaround time between batches or even when switching processes.

This invention enables the application of single use bioreactors for the culture of bacteria, yeasts and fungi by improving the transfer of one or more of the constituents of gaseous mixture to the system. This is done increasing the kLa and gas transfer rate using a micro-bubble generating system coupled to the agitation means, independent of it or both integrated.

### Description of the Invention

As stated previously, a limiting factor preventing the wider use of SUB is their low oxygen transfer capability. In bioreactors, oxygen transfer capabilities are related to the volumetric oxygen mass transfer coefficient (kLa) that can be viewed as the inverse of the resistance to oxygen transfer. Moreover, the kLa is the combination of two terms: the specific mass transfer coefficient (kL) and the specific phase exchange area (a). For a given system, the kLa is a complex function of the agitation speed, the gas flow and the viscosity of the media. All other things held constant, an increase of either term of the kLa yields an increase in the oxygen transfer rate.

Our approach consists on modifying both terms of the kLa by means of:
a) Introducing the air into the reactor in the form of microbubbles, and
b) Optimizing the agitation configuration to account for the use of microbubbles.

In addition it has been discovered a surprising effect to achieve industrially relevant cell densities when improving heat removal in SUB. This is done by adding an external broth recirculation loop containing a single use heat exchanger coupled with a droplet/microdrops formation system in the bioreactor.

Therefore a) and b) could be potentially combined with the use of an external recirculation loop where a thin film of returning liquid trickles through sinterized metal elements were microbubbles are generated.

The bioreactor where this system is implemented is made of a biocompatible plastic material, provides a controlled environment comparable to that of stainless steel bioreactors. The bioreactor is sealed and includes an agitation shaft, a microbubble generation system

In some embodiments, the coupling of the agitation shaft takes place by means of a mechanical seal located on the top section of the SUB whereas in some other embodiments the seal is located in the bottom section of the SUB.

In some embodiments, the microbubble generators are integrated to the agitation shaft, which has an internal conduit for the gas. In some other embodiments the microbubble generators are located on the bottom section of the SUB and are independent of the agitation shaft. Yet in other embodiments both systems are present at the same time.

In some embodiments there is an external recirculation loop where the culture media is returned to the top section of the reactor where it trickles on top of one or more microbubble generators.

### Preferred Description of the Invention

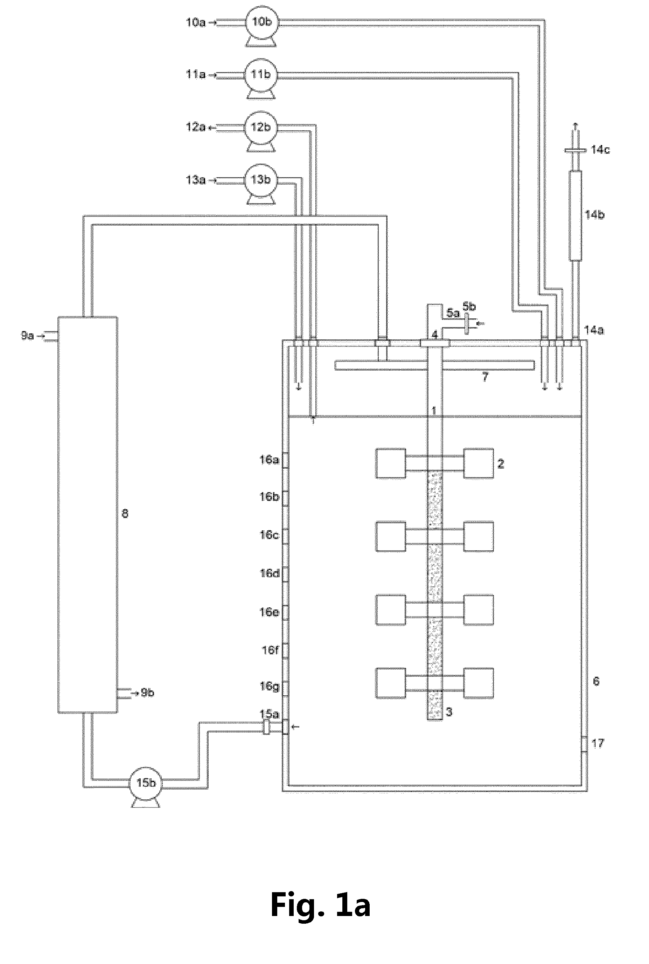

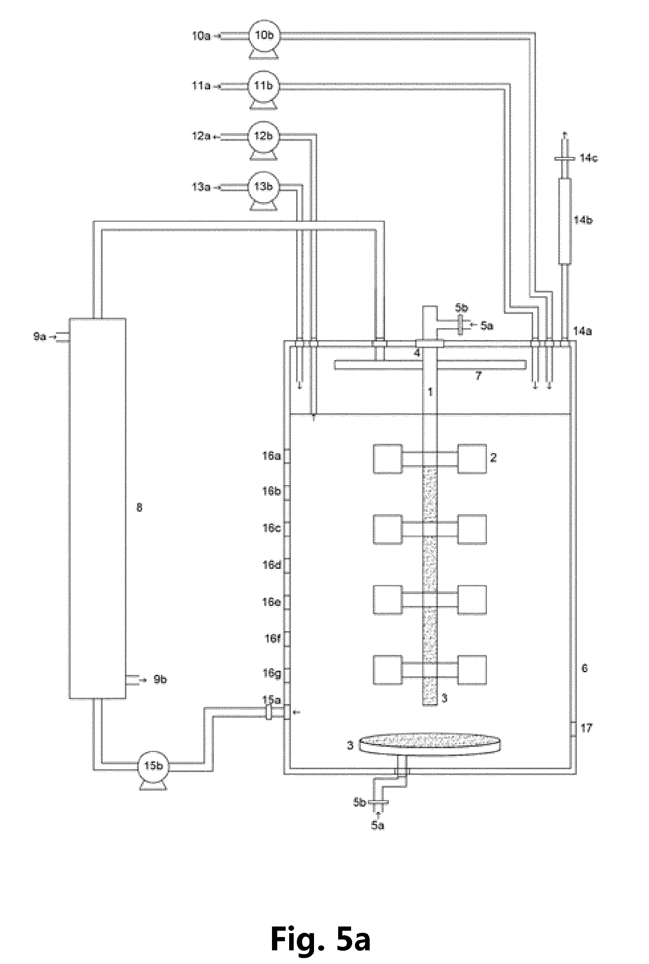

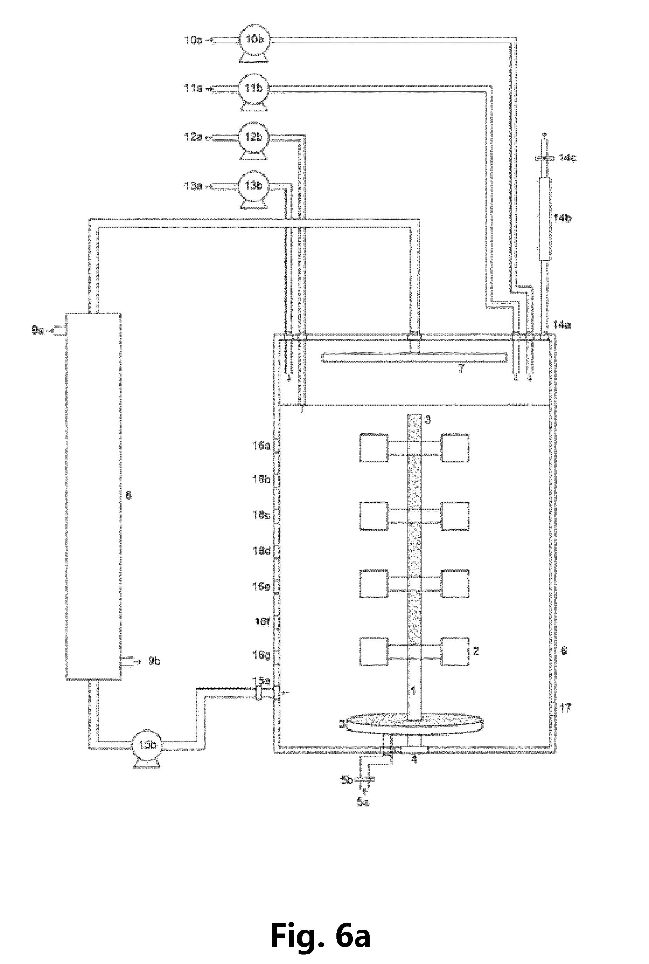

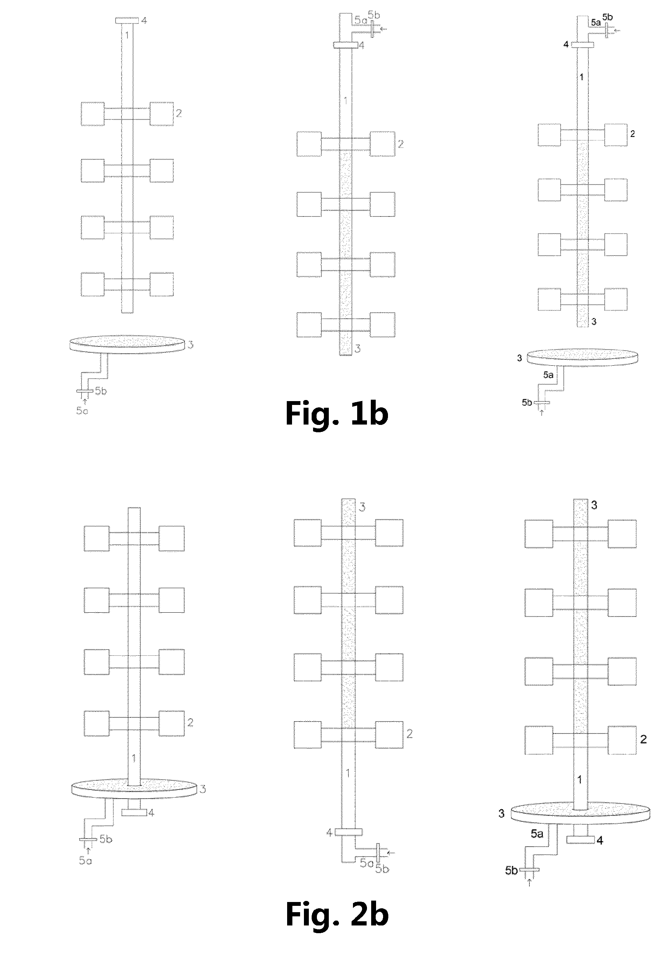

The agitation-aeration system according to one of the embodiments presented in the drawings comprises:
1. A closed cylindrical vessel, made of plastic material, whose characteristics can be seen in Figures 1a, 2a, 3a, 4a, 5a and 6a. The bioreactor is of single use and, once filled with the culture liquids through a sterile transfer system, can be handled and transported through a non-sterile environment for the duration of the process
2. An agitation-aeration system, shown in Figures 1b, 2b and 3b consisting of a mechanical agitator element, driven by an electric motor with input at the top or bottom of the bioreactor having in its interior a conduit for gases, with gas outlet through a) sintered metal cylinders for generation of microbubbles or b) a sintered metal plate at the bioreactor base or c) both systems (a) and (b) mounted in the same bioreactor system.
3. A system of access points (ports) for devices that respecting the sterile barrier, enables the automatic monitoring and control of cultivation parameters.
   In a preferred embodiment the single use bioreactor has a volume between 2 and 2000 liters.

### Description of the Agitation-Aeration system

The bioreactor has an agitation shaft driven by an electric motor. This shaft enters the reactor though the top or the bottom of the vessel and has an internal conduit for gases with the gas outlets being sintered metal cylinders for the generation of microbubbles (drawings 1b and 2b), with pore sizes ranging from 0,5 microns up to 1 mm.

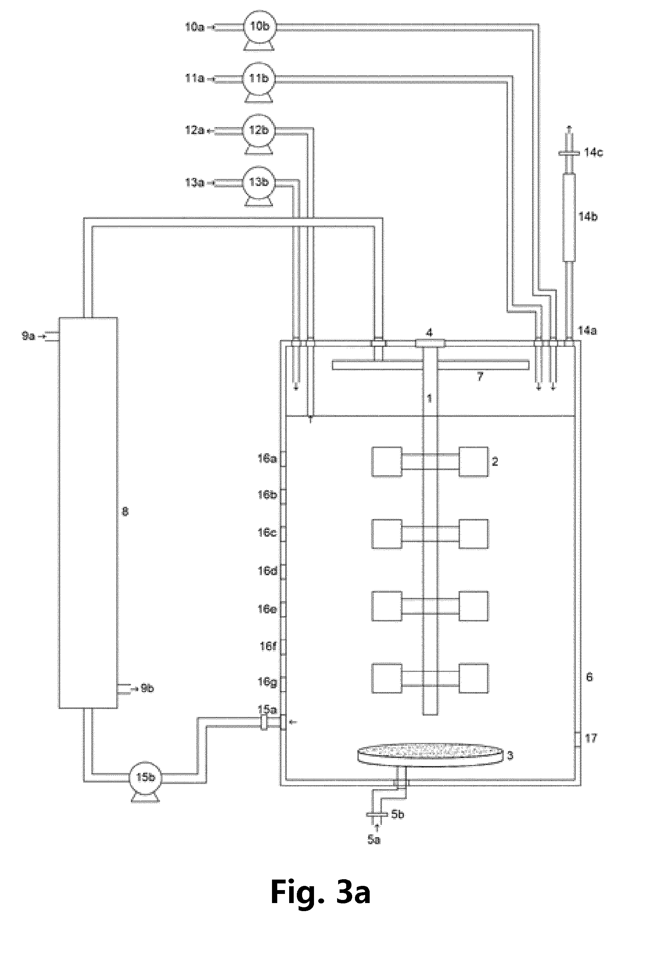

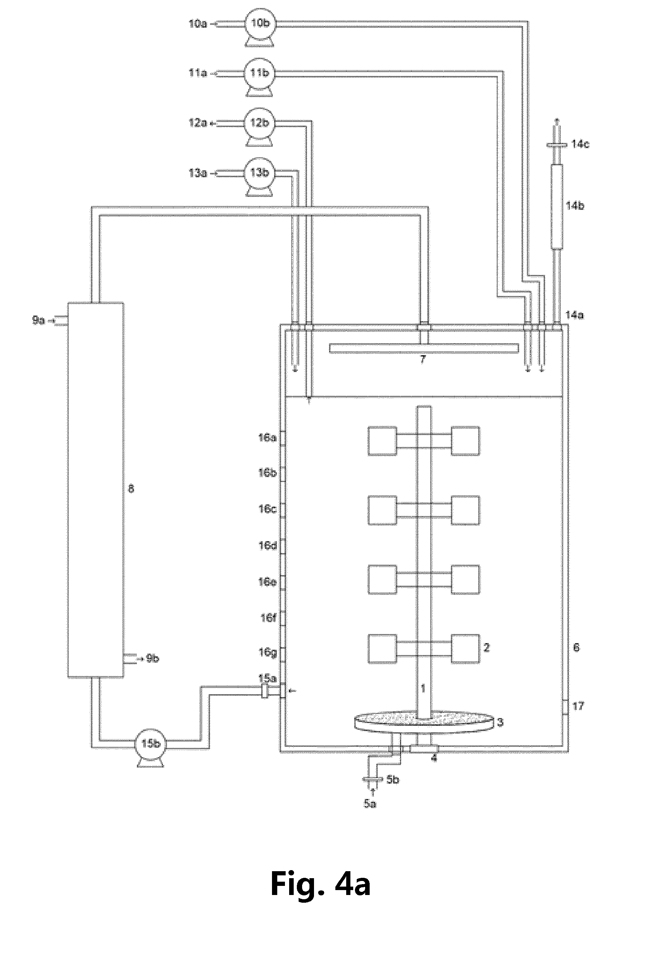

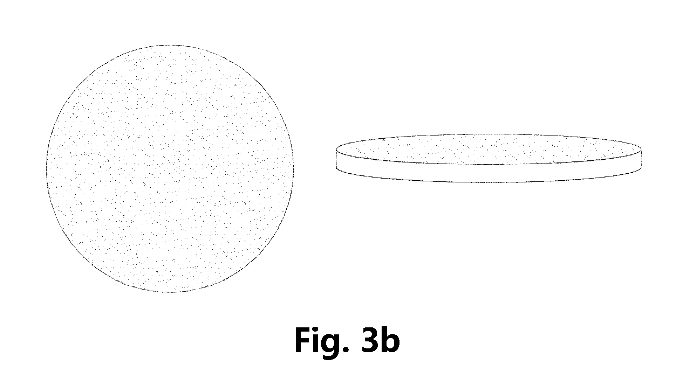

Alternatively, the bioreactor has an agitation shaft without the internal gas conduit, is driven by an electric motor and there is a sintered metal plate at the base of the reactor to create microbubbles (figure 3b and 4b) with pore sizes ranging from 0,5 microns up to 1 mm.

Alternatively, the bioreactor has an agitation shaft driven by an electric motor. This shaft enters the reactor though the top or the bottom and has an internal conduit for gases with the gas outlets being sintered metal cylinders for the generation of microbubbles with pore sizes ranging from 0,5 microns up to 1 mm. There is also a sintered metal plate at the base of the reactor to create microbubbles (figure 1b, 2b and 3b) with pore sizes ranging from 0,5 microns up to 1 mm.

### Description of the External Recirculation Heat Exchanger Sytem-Percolation.

Figures 1a, 2a, 3a, 4a, 5a and 6a show a vertical section of the single use system for energy exchange and droplet generation located in the external recirculation loop.

### Description of the Elements for Optical Ports and External Connection Systems.

The bioreactor has several optical ports for performing optical measurements based on fluorimetry, which always remain submerged below the liquid level.

The bioreactor also has inlet and outlet gas ports, ports for filling, inoculating and the occasional supply and drainage of fluids, an optional port (initially not perforated) for the use of non-optical probes or microprobes, and an agitation shaft.

Figures 1a, 2a, 3a, 4a, 5a and 6a allow to pinpoint the location of the heat exchange through an external recirculation loop, power to the agitation shaft, as well as port for sensors (optical or not) and microsensors.

The bioreactor will also have gas inlet and outlet ports to provide adequate partial pressure of oxygen to the culture, as well as other gases as necessary. Sterility of the gaseous streams is ensured through filters connected to those ports.

The filling, inoculation and sampling of the bioreactor is carried out through one or more dedicated ports. For continuous or fed-batch operations, one or more of these ports could be used for the addition or removal of liquids as needed.

Measurements of the culture parameters will be conducted externally, without interfering with the sterile barrier of the bioreactor. Possible measurement methods to be used are:
- Optical access to the contents of each bioreactor, from transparent walls or parts thereof.
- Access to the external pins of sensors or microsensors located on the inside of the bioreactors.

Heat will be added or removed through the support of the bioreactor system and/or through the external recirculation system to achieve the thermal homogeneity of the system.

The bioreactor as a whole will be disposable and will be presented in a sealed envelope or bag. It will be sterilized by gamma radiation and the filling and/or, inoculation operations as well as the connection of filtration systems and monitoring elements to the reactor will not require specific tools. For the final user, the bioreactor will be a consumable item.

The monitoring elements can be connected to a Digital Control Unit (DCU) allowing the automatic control of operational variables of the system. The control management system will monitor the evolution of the culture parameters and their response and act over the control setpoints if needed.

### Brief Description of the Drawings

For a better understanding of what has been disclosed, we present some drawings that schematically and in a non-limiting way exemplify specific embodiments of the invention.

Figures 1a, 2a, 3a, 4a, 5a and 6a are vertical sections of several embodiments of the bioreactor according to the invention, where:
1 agitation shaft
2 stirring paddle
3 microbubble generation system
4 mechanical seal
5a-b gas inlet pipe
6 plastic bag bioreactor
7 percolation system
8 external heat exchange device
9 a-b cooling-heating piping
10a, 11a, 12a, 13a ports for the introduction, withdrawal and return of liquids
10b, 11 b, 12b, 13b peristaltic pumps
14 a-b-c gas exhaustion outlet and condenser
15a-b peristaltic pump and piping
16 (a-g) optical and invasive sensor ports
17 temperature sensor port

Fig. 1a plan view of an embodiment with an agitator shaft, driven by an electric motor, entering from the top of the bioreactor. This shaft has an internal conduit for gases, with gas outlet through sintered metal cylinders for microbubble generation;
Fig. 2a plan view of an embodiment with an agitator shaft, driven by an electric motor, entering from the bottom of the bioreactor. This shaft has an internal conduit for gases, with gas outlet through sintered metal cylinders for microbubble generation;
Fig. 3a plan view of an embodiment with an agitator shaft, driven by an electric motor, entering from the top of the bioreactor with a sintered metal plate at the bottom of the reactor to create microbubbles;
Fig. 4a plan view of an embodiment with an agitator shaft, driven by an electric motor, entering from the bottom of the bioreactor with a sintered metal plate at the bottom of the reactor to create microbubbles;
Fig. 5a plan view of an embodiment with an agitator shaft, driven by an electric motor, entering from the top of the bioreactor. This shaft has an internal conduit for gases, with gas outlet through sintered metal cylinders for microbubble generation. This embodiment also features a sintered metal plate at the bottom of the reactor to create microbubbles;
Fig. 6a plan view of an embodiment with an agitator shaft, driven by an electric motor, entering from the bottom of the bioreactor. This shaft has an internal conduit for gases, with gas outlet through sintered metal cylinders for microbubble generation. This embodiment also features a sintered metal plate at the bottom of the reactor to create microbubbles;

Fig. 1b shows vertical sectional views of the agitation system coupled to the stirring shaft through the top.

Left: Agitation shaft without aeration and sintered metal plate at the bottom of the reactor to create microbubbles.

Centre: Shaft with internal conduit for gases, with gas outlet through sintered metal cylinders for microbubble generation.

Right: Shaft with internal conduit for gases, with gas outlet through sintered metal cylinders for microbubble generation and with a sintered metal plate at the bottom of the reactor to create microbubbles;

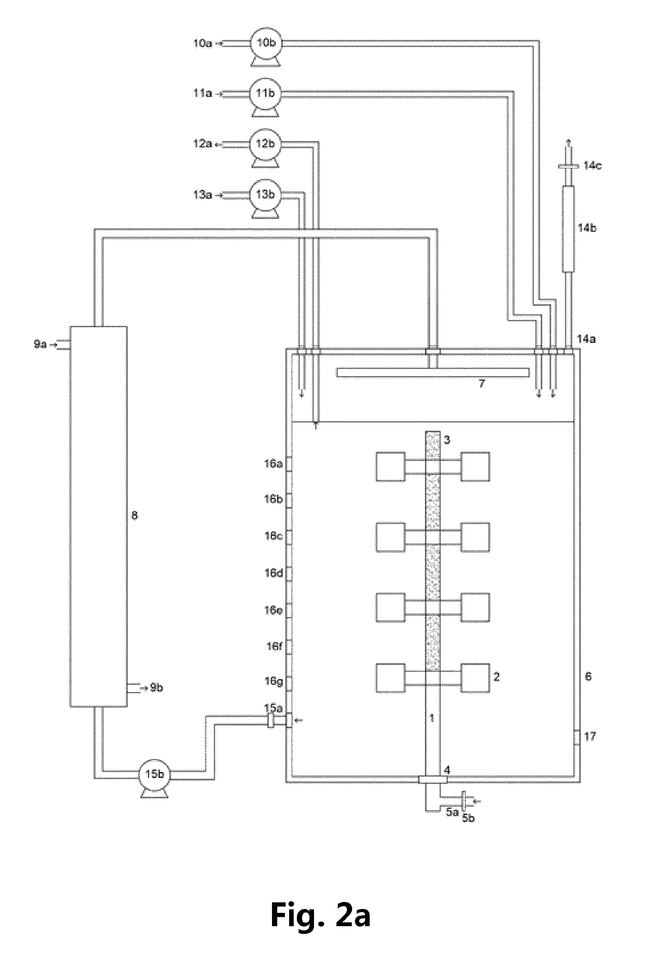

Fig. 2b different plan views of all the agitation system embodiments where the with an stirring shaft entering through the bottom of the reactor:
Left: Agitation shaft without air conduit and sintered metal plate at the bottom of the reactor for generating microbubbles.
Centre: Shaft with internal conduit for gases, with gas outlet through sintered metal cylinders for microbubble generation.
Right: Shaft with internal conduit for gases, with gas outlet through sintered metal cylinders for microbubble generation and with a sintered metal plate at the bottom of the reactor to create microbubbles; and

Fig. 3b Detail of the sintered metal plate located at the bottom of the reactor to create microbubbles.

## Claims

1. Device suitable for a single use bioreactor (SUB) **characterized in that** it comprises :
a) Microbubble generation system, and
b) Agitation means

2. The device of claim 1, wherein the agitation means is a mechanical shaft.

3. The device of claim 2, wherein the microbubble generation system is integrated in the shaft.

4. The device of claim 1, wherein the microbubble generation system is a disc with pores.

5. The device of any of the previous claims, wherein the pore sizes of the microbubble generation system range from 0,5 microns up to 1 mm.

6. The device of any of the previous claims, further comprising an external recirculation loop device suitable for a single use bioreactor (SUB) **characterized in that** it comprises:
a) Single-use external heat exchange device.
b) Single-use droplet/microdrops formation device (percolation) located in the path of the returning fluid to the reactor head space.
c) Peristaltic pump.

7. Single use bioreactor **characterized in that** it comprises a device according any of the claims 1-6.
